# EUROPEAN PATENT SPECIFICATION

(11) **EP 2 300 492 B1**
(45) Date of publication and mention of the grant of the patent: **04.06.2014**
(21) Application number: 09762696.4
(22) Date of filing: 10.06.2009
(51) Int. Cl.: C07K 7/06, A61K 38/00, A61K 38/08, A61K 39/145, A61P 31/16, C07K 14/11, C12N 7/01, C12N 15/44, C12N 15/62, C12Q 1/70, G01N 33/53

(54) **A NOVEL ANTIVIRAL PEPTIDE AGAINST AVIAN INFLUENZA VIRUS H9N2**
NEUES ANTIVIRALES PEPTID GEGEN DAS VOGELGRIPPEVIRUS H9N2
NOUVEAU PEPTIDE ANTIVIRAL CONTRE LE VIRUS DE LA GRIPPE AVIAIRE H9N2

(30) Priority: 12.06.2008 MY 0802061
(43) Date of publication of application: 30.03.2011
(73) Proprietor: Universiti Putra Malaysia, 43400 UPM Serdang Selangor Darul Ehsan (MY)
(72) Inventor: YUSOFF, Khatijah, Mohd, 43400 UPM Serdang (MY); OMAR, Abdul, Rahman, 43400 UPM Serdang (MY); IDERIS, Aini, 43400 UPM Serdang (MY); RAJIK, Mohamed, 43400 UPM Serdang (MY)
(74) Representative: Robertson, James Alexander
(86) International application number: PCT/MY2009/000071
(87) International publication number: WO 2009/151313

(56) References cited:
- EP-A1- 1 167 382
- WO-A1-2007/105565
- WO-A1-2008/154813
- WO-A2-99/47656
- WO-A2-03/006620
- WO-A2-2005/084190
- WO-A2-2008/006028
- WO-A2-2008/157419
- CRUTZ-LE COQ A-M ET AL: "Sequence analysis of the lactococcal bacteriophage bIL170: Insights into structural proteins and HNH endonucleases in dairy phages", MICROBIOLOGY, SOCIETY FOR GENERAL MICROBIOLOGY, READING, GB, vol. 148, no. 4, 1 April 2002 (2002-04-01) , pages 985-1001, XP002465569, ISSN: 1350-0872
- KUANG, Z. ET AL.: 'Screening of peptides as broad-spectrum neuraminidase inhibitors against influenza viruses' BING DU XUE BAO. vol. 23, no. 3, 2007, pages 165 - 171, XP008140645
- SONG, H. ET AL.: 'Identification of peptide mimotopes of an abroad-spectrum neutralizing epitope of highly pathogenic avian influenza hemagglutinin' BING DU XUE BAO. vol. 24, no. 6, November 2008, pages 421 - 426, XP008140795
- RAJIK, M. ET AL.: 'Identification and characterisation of a novel anti-viral peptide against avian influenza virus H9N2' VIROLOGY JOURNAL. vol. 6, no. 74, 05 June 2009, pages 1 - 12, XP021059691
- RAJIK, M. ET AL.: 'A novel peptide inhibits the influenza virus replication by preventing the viral attachment to the host cells' INTERNATIONAL JOURNAL OF BIOLOGICAL SCIENCES. vol. 5, no. 6, 08 August 2009, pages 543 - 548, XP008140577

## Description

### 1.0 FIELD OF INVENTION

The present invention relates to a novel antiviral peptide and a fusion phage which acts against Avian Influenza Virus (AIV) subtype H₉N₂. More specifically, the fusion phage displays the sequence CNDFRSKTC on its surface protein P₃ which binds to AIV H₉N₂ with an IC₅₀ value of less 10¹³ pfu/100 µl. Synthetic peptides with amino acid sequence CNDFRSKTC, either in linear or cyclic conformations, inhibited the propagation of AIV H₉N₂ with an IC₅₀ value less than about 100 µM.

### 2.0 BACKGROUND OF INVENTION

Avian influenza virus (AIV) belongs to the family of *Orthomyxoviridae* which contains two genera, influenza A & B and influenza C (Lamb and Krug, 1996). These viruses are the major cause of morbidity and mortality among poultries in the world. It is the causative agent of the most dangerous disease, called bird flu in common terms (Webster *et* al.,, 1992). Although these viruses do not infect humans, several instances of human infections and outbreaks have been reported (CDC, 2008; Normile, 2004; Parry, 2004). Influenza A viruses are enveloped with lipid bilayer and contain eight single-stranded, segmented, negative sense RNAs. There are two glycoproteins present on the surface of the virions namely, haemagglutinin (HA) and neurammidase (NA), and one ion channel protein (M2). The glycoproteins are the major antigenic determinants of influenza viruses. The HA protein initiates the first step in the viral infection, which involves the attachment of viruses to the host cell surface sialic acid receptors (Lamb and Krug, 1996). The NA protein participates in the release of mature virions from the host cells (Palese *ef al.,* 1974). Therefore, in order to study the virus - host interaction and also to identify molecules that inhibit this process a bacteriophage displaying a specific peptide sequence was selected by its affinity to avian influenza virus strain H₉N₂ using a phage display library.

The preferred primary strategy for the prevention of influenza virus infection is annual vaccination among susceptible population. But the antiviral drugs play an important role in a comprehensive approach to control the illness and transmission (Hayden, 2006). There are two classes of antiviral drugs that have been approved for the treatment and prophylaxis. They are adamantane derivatives (amantadine and rimantadine) and neuraminidase inhibitors (NAls; zanarnivir and oseltarnivir) (Nicholson *et* al., 2003). These adamantane derivatives act by binding and blocking the function of influenza A virus M2 ion channel protein and thereby prevents the viral replication inside the host cell (Wang *et* al., 1993)). Due to single point mutations in M2 proteins, adamantane resistant strains have emerged (Hay et al., 1986). These resistant viruses are typically fully pathogenic and transmissible (Hayden, 2006). The NAN inhibit the enzymatic activity of the neuraminidase protein and prevents viral release from the infected host cell. But NAI resistant strains have also emerged due to the mutations in the active site of the NA (Nicholson *et* al., 2003). The increasing resistance by the influenza A viruses against the both types of drugs highlights our necessity to identify novel drugs.

WO 2008/154813 discloses antibodies that specifically bind to the hemagglutinin (HA) of avian influenza virus subtype H5. Such monoclonal antibodies are useful in the detection, diagnosis, prevention and treatment of avian influenza virus, especially avian influenza virus subtype H5.

EP1167382 discloses the identification of a peptide that binds to haemagglutinin (HA) of the influenza virus. The peptide binds specifically to the hemagglutinin associated with the first step of influenza virus infection to prevent binding of the virus to the host receptor.

WO 2007/205565 discloses a peptide with high affinity to haemagglutinin and an inhibitory activity on the infection by influenza virus.

Traditionally, compounds from natural products obtained from plants, marine organisms, fungi or other microorganisms are used to identify antimicrobial or antiviral agents. Recently, combinatorial peptide libraries like phage display library are increasingly being used to identify peptide compounds for the same purpose (Doorbar *et* al., 1994). The broad structural diversity of peptides displayed on bacteriophages has made the phage display library an important tool to study proteinprotein interactions, especially in the identification of specific ligands that interact with a particular target (Devlin *et* al.,1990). Compounds that interact with target molecules are selected from phage libraries and screening processes are then used to identify lead compounds that have functional effects on the target. These lead compounds are then optimized for their activity and then the candidate drugs enter into clinical trials.

We used a cyclic peptide phage display library to identify peptide molecule that interacts with the influenza A virus H9N2 and proved its antiviral property in *vitro* and in *ovo*.

### 3.0 SUMMARY OF THE INVENTION

Accordingly it is an object of the present invention to provide for novel peptides and fusion phages that has antiviral activity against avian influenza virus A H₉N₂.

It is another object of the present invention to provide for a pharmaceutical composition containing a recombinant phage or a synthetic peptide according to the invention.

It is yet another object of the present invention to provide a diagnostic reagent containing a recombinant phage or a synthetic peptide according to the invention.

These objectives are accomplished by,

An isolated and purified recombinant phage bearing a fusion peptide or synthetic peptides derived from the fusion phage that binds to avian influenza virus A H₉N₂ and inhibits the propagation of the virus.

To identify a novel antiviral molecule, a population of recombinant phages displaying random disulfide constrained heptapeptide sequences was screened against the virus. Then the peptide displayed on the surface of the fusion phage was synthesized chemically. The isolated peptide has the sequence of NDFRSKT with an IC₅₀ value of less than 100 µm. The specificity of the recombinant phage against the influenza virus A H₉N₂ was proved by an antibody - phage competition assay, in which the phages were able to compete with polyclonal antibodies for binding site on the viral surface proteins.

In the present invention, synthetic peptides based upon one of the isolated sequences, either in linear or cyclic conformation, such as the synthetic peptide CNDFRSKTC, are able to inhibit the propagation of NDV, thereby preventing disease and spread of infection.

### 4.0 BRIEF DESCRIPTION OF THE FIGURES AND TABLE

Table I shows the heptapeptide sequences obtained from three rounds of biopanning against AIV H₉N₂.
Table 2 shows haernagglutination inhibition activities of synthetic peptides and fusion phages.

Figure I shows the results of the competition between recombinant phages bearing specific heptapeptide sequence and polyclonal antibodies for AIV H9N2.
Figure 2 shows the inhibition of AIV propagation with synthetic peptides in *ovo*.
Figure 3 shows the inhibition of AIV propagation with fusion phages *in ovo*.
Figure 4 shows the inhibition of AIV propagation with fusion phages *in vitro.*
Figure 5 shows the effect of fusion phage on viral replication *in vitro*.

### 5.0 DETAILED DESCRIPTION OF THE TABLES AND FIGURES

Table I shows the heptapeptide sequences obtained from four rounds of biopanning against AIV. After 4 rounds of selection and amplification 20, 35 and 35 individual clones from the 2^{nd}, 3^{rd} and 4^{th} rounds, respectively, were sequenced.
Table 2 shows the inhibitory ability of the cyclic and linear peptides against the haernaggluti nation activity of the avian influenza virus H₉N₂. Experiments were performed in triplicates. + in the presence of, - in the absence of.

Figure I shows the results of the competition between recombinant phages bearing specific heptapeptide sequence and polyclonal antibodies for AIV. The polyclonal antibodies (pAb) inhibited the association of the fusion phage with AIV, suggesting that the phages may share some common binding sites with the antibody. Experiments were done in triplicates and the error bars represent the standard deviation of the mean. Series I - Fusion phage; Series 2 - Fusion phage with polyclonal Antibodies.

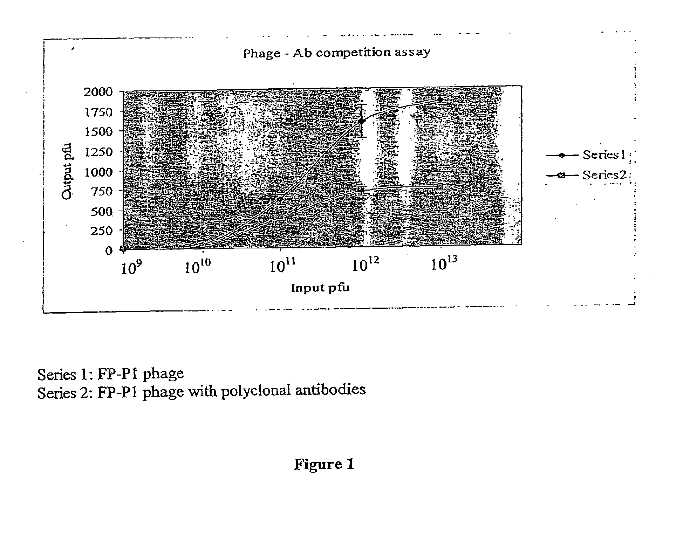

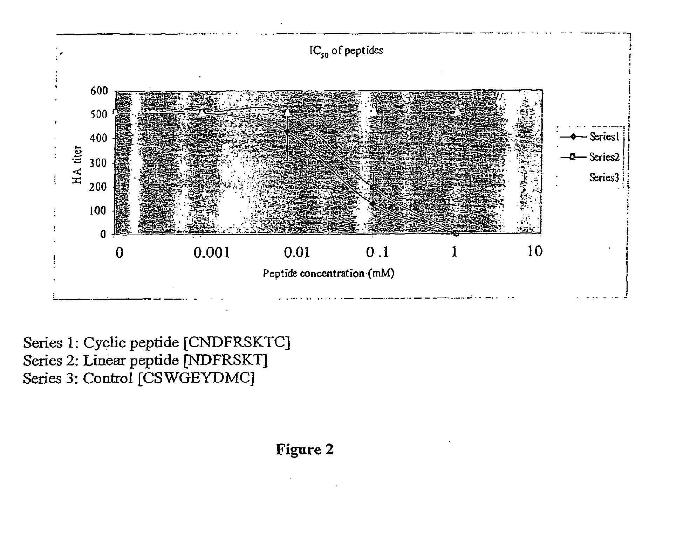

Figure 2 shows the results of determination of the IC₅₀ values of the synthetic peptides in ovo against AIV H₉N₂ propagation. Peptide concentration needed to inhibit 50% of the virus growth was determined using different concentrations of peptides. Experiments were done in triplicates and the error bars represent the standard deviation of the mean.

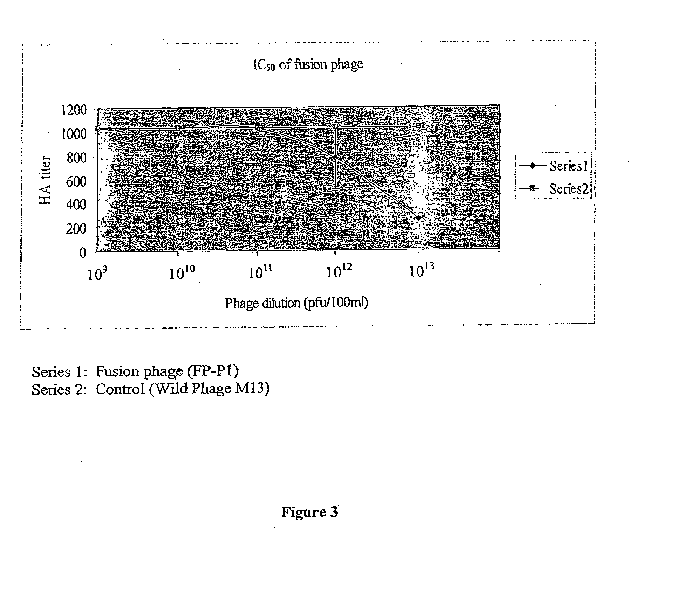

Figure 3 shows the results Of IC₅₀ values of fusion phage FP-P1 against AIV H₉N₂ virus propagation in ovo. Experiments were done in triplicates and the error bars represent the standard deviation of the mean. Series I - Fusion phage FP-Pl; Series 2 - Wild phage as control.

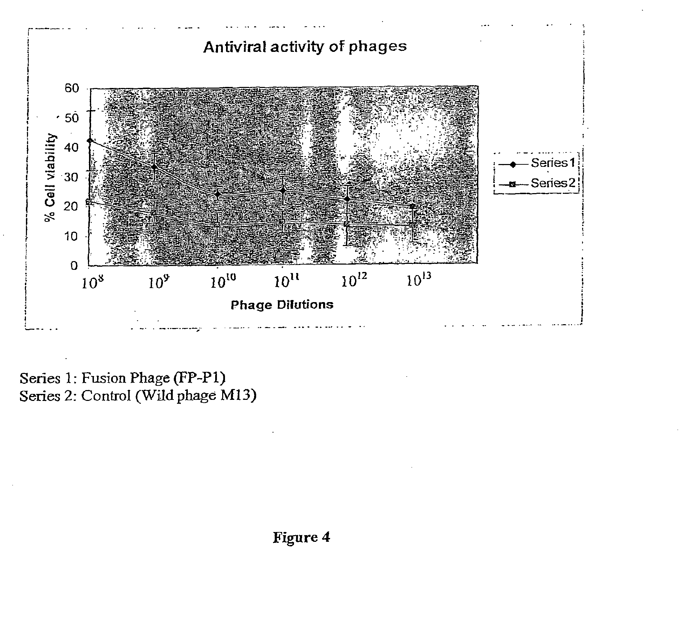

Figure 4 shows the results of antiviral activity of the fusion phage FP-P1 in *vitro.* Experiments were done in triplicates and the error bars represent the standard deviation of the mean. Series I - FP-P 1; Series 2 - Wild Phage M 13 as control.

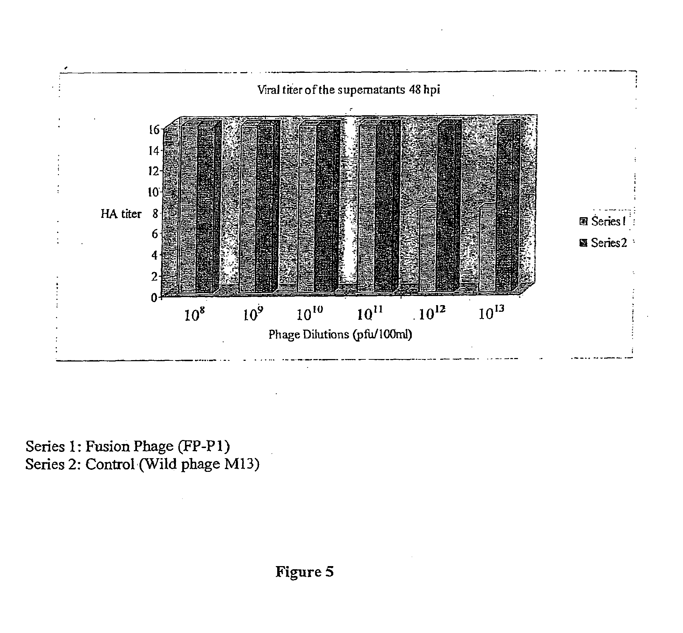

Figure 5 shows the results of the effect of the fusion phage FP-P1 on viral replication. MDCK cells were inoculated with untreated (o value) or FP-P I treated virus, and viral titers were determined on supernatants 72 hpi. Series I - FP-Pl; Series 2 - Wild phage M 13 as control.

### 6.0 DETAILED DESCRIPTION OF THE INVENTION

The description described herein will be more fully understood with the following detailed description which is divided into different sections. Materials and methods employed in this invention are set forth in Examples. Examples I to 5 are the conventional materials and methods which are prerequisites of the invention.

### Principles of phage display technology

In phage display technology, random peptides are usually displayed on the surface of bacteriophage molecules as fusion protein by inserting synthetic oligonucleotides in the gene encoding the coat proteins. A collection of these recombinant phages are known as phage display library, allows the screening of vast numbers of peptide sequences against a target by an in *vitro* selection procedure known as biopanning (Parmley *et al.,* 1988 and Smith *et al.,* 1993). In the present invention, the target used was whole AIV H₉N₂ virus particles and the library employed was the disulfide constrained library obtained from New England Biolabs, Inc. In this particular library, the displayed peptide molecules are flanked by a cysteine residue to the gpIII protein of phage M13. The cysteine residues help the peptides to attain a fixed cyclic shape, in the absence of reducing agents. This disulfide constrained library useful for targets whose interacting components (native ligands) have discontinuous binding sites (conformational epitope) (Hoess *et al.,* 1994); in which amino acids are brought from different positions in a polypeptide to form an essential contact area. Besides, the disulfide constrained cyclic peptide library is more useful in selecting high affinity ligands rather than a linear peptide library (O'Neil *et aL,,* 1992; Gho *et al.,* 1997).

### Affinity selection of peptides that bind to AIV

The avian influenza virus contains two surface glycoproteins namely haernagglutinin (HA) and neuraminidase (NA) which is responsible for virus entry and exit into and from the host cell. We therefore decided to employ the disulfide constrained library against the whole virus particles to select for conformational ligands which bind the surface proteins. Figure I summarizes the major steps involved in the selection of these ligands. First, the whole virus particles were directly attached to the surface of a high binding microtiter plate well. The library was then added into the well to allow the recombinant phage particles to bind with the virus particles. The unbound phages were washed out and the bound phages were eluted at low pH. The eluted phages were then amplified in the bacterium, *Escherichia coli* and the amplified phages were used in second round of biopanning. This procedure was repeated for four times. After four successive rounds of affinity selection and amplification, a subset of the selected phages was grown up individually and tile identity of the peptides that bind to core particles were obtained by sequencing tile gpIII gene carrying the insertion.

In order to select high affinity binding phages; the stringency of selection was increased by (i) performing the biopanning at room temperature (28'C), (ii) shortening the time of binding to I h to select for ligands with rapid on rates (Ko,), (iii) washing the wells thoroughly (10 times) to remove low affinity binding phages, and (iv) repeating 4 rounds of biopanning to enrich high affinity binding clones. 47% of phages analysed from the fourth round panning carried the fusion peptide sequence NDFRSKT, 10.5% containing QHSTKWF motif followed by LPYAAKH and ILGDKVG, 5% each and other unrelated sequences (Table 1). Streptavidin target was used for positive control which gave a consensus motif of HPQ sequence in all clones, which in good agreement with that reported by Devlin et al. (1990). No recognizable consensus sequence was observed with bovine serum albumin (BSA), which was used as negative control.

As used herein, the term "fusion peptide" refers to amino acid sequence genetically encoded by a bacteriophage and physically linked to a coat protein of the phage. The claimed fusion peptides contain amino acid sequences NDFRSKT, but it is not limited to: (i) amino acid sequence which is shorter or longer than the claimed amino acid sequences; (ii) variations in the amino acid sequences, particularly amino acid substitutions within the same category as described below; (iii) amino acid sequences sharing at least 60% homology with those of the claimed fusion peptides and; (iv) either linear or constrained conformation.

AIV possesses two surface glycoproteins, HA and NA, which protrude from the viral lipid bilayer membrane. These glycoproteins are essential for the entry and release of viruses into and outside of the host cells respectively. Since the phages bearing the sequences NDFRSKT bound to the virion in solution, these sequences may, to a certain extent, resemble a region on the host cell receptor that interacts with the intact virion.

### Phages compete with antibody for binding sites on AIV

The surface glycoproteins HA and NA are two important proteins that elicit antibody production in the host. In a phage - antibody competition assay, the phages were able to bind to one of the mimotopes and therefore inhibit the binding of antibodies raised against the virion. Figure I shows that the recombinant phages displaying the peptide NDFRSKT, selected from biopanning experiment, were able to compete with the antibodies for the binding site on AIV. In the presence of the antibody, the number of phages bound to the virus coated wells reduced dramatically as a result of competition between these two molecules for the same binding site on the virus. Thus the recombinant phage molecules may serve as a potential diagnostic agent for the AIV infection.

### Antiviral activity of synthetic peptides and fusion phages in ovo

AIV can easily be propagated in the allantoic fluid of embryonated chicken eggs. Therefore, the virus and the propagation system provide a simple model to assess directly the antiviral activities of a particular compound or peptide in vivo. The amount of virus present in the allantoic fluids, before and after the antiviral compounds treatment, is determined by haernagglutination titer. Injection of the claimed peptide either in cyclic or linear form and the fusion phage displaying the peptide reduced the AIV titer in the allantoic fluid (Figures 2 and 3). The cyclic peptide showed higher IC₅₀ value at a peptide concentration lower than 100 µM to the linear peptide. Furthermore, cyclization can avoid the digestion of the peptides by exopeptidases. Thus the synthetic peptides claimed herein can be used as therapeutic agents to treat, control and also to eradicate the disease.

### Antiviral activity of fusion phages in vitro

MDCK cell lines are effective medium for the propagation of avian influenza viruses in *vitro.* The in vitro antiviral property of the fusion phages against the virus multiplication were checked in these cell lines. The inhibition of viral replication was indirectly checked by the cell viability, before and after the treatment with the fusion phage molecules. The cell viability increases nearly 2 fold after the infection with phage treated viruses to the untreated viruses (Figure 4). The reduction in viral titer was further confirmed by haemagglutination test of the supernatants (Figure 5). This experiment proved the antiviral property of the fusion phage molecules in *vitro.*

### EXAMPLES

### Example 1:

**Propagation and purification of AIV:** The propagation and purification methods were adapted and modified from Blaskovic and Styk (1967) and Yusoff *et al*. (1996), respectively. AIV subtype H₉N₂ was injected into a 9 days old specific pathogen free embryonated chicken eggs. After 3 days of incubation at 37°C, the allantoic fluid was harvested and clarified by centrifugation (30 min, 35,000 x g, 4°C. The virus was purified from the clarified supernatants by 30%-60% sucrose gradient ultracentrifugation (3.5 h, 285,000 x g, 4°C and was used for the studies below.

### Example 2:

**Biopanning of purified AIV:** The protein concentration of virus was measured using the Bradford assay (1976). AIV (15 µg/ml) was coated onto a microtiter plate well [Na₂CO₃/NaHCO₃ buffer (0.1 M, pH 9.6)]. Streptavidin and BSA were used as positive and negative controls respectively. The experiment was carried out at room temperature (about 25°C). Phages from a disulfide constrained *7-mer* phage display library (New England Biolabs, USA) were diluted to I X 10¹¹ pfu in TBS [50 mM Tris-HCI (pH 7.5), 150 mM NaCl; 110 µl] and added into each coated well. The mixtures were incubated for 30 min. The wells were washed with TBST JBS + 0.1% v/v Tween-20). Bound phages were eluted with glycine-HCI [0. 1 M, (pH 2.2); 100 µl] by rocking gently for 7-8 min and neutralized with Tris-HCI [I M, (pH 9); 115 µl. The output pfu was determined by titrating the eluate (10 µl) while the remainder eluate was amplified in E. coli stain ER2738 (F' Iacl^{q} *Δ(IacZ)M]₅proA⁺B⁺ zzf::TnIo(Tet^{R})*/*fhuA2supE thi Δ(lac-proAB) Δ(hsdMS-mcrB)5* (rₖ-mₖ-McrBC-)} at 37°C, shaking for 5 hr. The panning process was repeated for a further 3 rounds.

### Example 3:

**Phage titration:** Phage titration method was adapted from Sambrook *et al.* (1989).

### Example 4:

**Sequencing of the selected phages:** Single plaques from all 4 rounds of panning were picked from (Luria Broth) LB plates (used in output titration) and grown in LB media. Single stranded DNA of the phages was extracted as described by Sambrook *et al,* (1989). Sequencing of inserts was done using the ABI automated sequencer by First Base Laboratories Sdn Bhd, Kuala lumpur.

### Example 5:

**Antibody competition assay:** Wells were coated with AIV (15 µg/ml) and polyclonal antibodies (1:500 dilution; 100 µl) raised against AIV were added with a series of different phage concentrations (10⁹ - 10¹³ pfu; 100 µl). The mixtures were incubated for I hour and washed 6 X with Tris-buffered saline - Tween (TBST). Bound phages were eluted and titered as described above.

### Example 6:

**Determination Of IC₅₀ of peptides and fusion phages in the inhibition of AIV propagation in ovo:** Nine-day-old embryonated chicken eggs were inoculated with 100 µl of AIV (8 HA units) and an equal volume of different concentrations of peptide (0. 1 µM - I mM) or fusion phages (10⁸ - 10¹³ pfu). The eggs were incubated at 37°C. Three eggs were used for each peptide concentration. For the positive control, only AIV was inoculated and for the negative control, the virus was substituted by peptides or phages. Peptides (100 µl) were injected into appropriate eggs daily for another 3 days. A day after the 4^{th} injection, the eggs were chilled overnight and the allantoic fluid was harvested. The haemagglutination activities of the allantoic fluid samples were then determined according to Grace *et al,* (1973).

### Example 7:

**Determination Of IC₅₀ of fusion phages in the inhibition of AIV propagation in *vitro*:** MDCK cells were inoculated with medium alone or phage treated (10⁸ - 1013 pfu / 100 µl) or non-treated virus (multiplicity of infection [MoI] 0.05) for I hr at 37°C. Following adsorption, monolayers were washed and incubated in EMEM containing 5% fetal bovine serum (FBS). Cytopathic effect was monitored by light microscopy and quantitated by XTT Cell Viability Assay Kit (Biotium, USA). To assess viral replication, MDCK cells were inoculated with phage-treated or untreated virus at an MOI of 0.05, supernatants were collected at 72 hpi, and viral titers were determined by HA titer. The IC₅₀ value was estimated by interpolation of the dose-response curve.

Although the invention has been described above with respect to various presently preferred embodiments, it will be apparent to one of ordinary skill in the art that many variations and modifications may be made. Therefore, the invention is not to be understood as limited to the particular embodiments presented herein but, rather, is to be understood as embracing all such variations and modifications which fall within the scope of the claims appended hereto.

**Table 1**

| Rounds of panning | Heptapeptide sequences | Frequency of sequences (%) |
|---|---|---|
| 2^{nd} round | Unrelated sequences | 100 |
| 3^{rd} round | LPYAAKH/LPYGSKH | 25 |
| | ILGYKVG | 17 |
| | Unrelated sequences | 58 |
| 4^{th} round | NDFRSKT | 47 |
| | QHSTKWF | 10.5 |
| | LPYAAKH | 5 |
| | ILGDKVG | 5 |
| | Unrelated sequences | 23 |
| 3^{rd} round | HPQFLSL | 55 |
| Streptavidin | GLYNHPQ | 27 |
| | Unrelated sequences | 18 |

**Table 2**

| Peptide concentration (mM; 50 µl) / Phage Particles | Agglutination of RBC (+ AIV 32 HAU; 50 µl) | Agglutination of RBC (- AIV 32 HAU; 50 µl) |
|---|---|---|
| Cyclic 1.0 | No | No |
| Cyclic 0.1 | No | No |
| Cyclic 0.01 | Yes | No |
| Cyclic 0 | Yes | No |
| Linear 1.0 | No | No |
| Linear 0.1 | No | No |
| Linear 0.01 | Yes | No |
| Linear 0 | Yes | No |
| Phage (10¹³ pfu / 100 µl) | No | No |
| Phage (10¹² pfu / 100 µl) | Yes | No |

## Claims

1. An isolated and purified recombinant phage bearing a fusion peptide which contains the amino acid sequence NDFRSKT and binds to Avian influenza virus subtype H9N2 and inhibits the propagation of the same.

2. A synthetic peptide which contains the sequence CNDFRSKTC or NDFRSKT and inhibits the propagation of avian influenza virus H9N2 by binding to the virus.

3. The synthetic peptide of claim 2 wherein said peptide has an IC₅₀ value less than 100 µm.

4. The synthetic peptide as claimed in any one of claims 2 or 3, which binds to the avian influenza virus H9N2 surface glycoproteins HA and NA.

5. A pharmaceutical composition containing a recombinant phage as claimed in claim 1.

6. A pharmaceutical composition containing a synthetic peptide as claimed in any one of claims 2-4.

7. A diagnostic reagent containing a recombinant phage as claimed in claim 1.

8. A diagnostic reagent containing a synthetic peptide as claimed in any one of claims 2-4.

9. A recombinant phage or synthetic peptide according to any of claims 1-4, for use in a method of treatment or diagnosis of avian flu.

10. The use of a recombinant phage or synthetic peptide according to any of claims 1-4 in the manufacture of a medicament for the treatment of avian flu.

## Patentansprüche

1. Isolierter und gereinigter rekombinanter Phage, der ein Fusionspeptid trägt, das die Aminosäuresequenz NDFRSKT enthält und an den Vogelgrippevirus-Subtyp H9N2 bindet und dessen Fortpflanzung hemmt.

2. Synthetisches Peptid, das die Sequenz CDNFRSKTC oder NDFRSKT trägt und die Fortpflanzung des Vogelgrippevirus H9N2 durch Binden an das Virus hemmt.

3. Synthetisches Peptid nach Anspruch 2, wobei das Peptid einen IC₅₀-Wert von weniger als 100 µm aufweist.

4. Synthetisches Peptid nach einem der Ansprüche 2 oder 3, das an die Oberflächenglycoproteine HA und NA des Vogelgrippevirus H9N2 bindet.

5. Pharmazeutische Zusammensetzung, die einen rekombinanten Phagen nach Anspruch 1 enthält.

6. Pharmazeutische Zusammensetzung, die ein synthetisches Peptid nach einem der Ansprüche 2-4 enthält.

7. Diagnostikum, das einen rekombinanten Phagen nach Anspruch 1 enthält.

8. Diagnostikum, das ein synthetisches Peptid nach einem der Ansprüche 2-4 enthält.

9. Rekombinanter Phage oder synthetisches Peptid nach einem der Ansprüche 1-4 zur Verwendung in einem Verfahren zur Behandlung oder Diagnose von Vogelgrippe.

10. Verwendung des rekombinanten Phagen oder synthetischen Peptids nach einem der Ansprüche 1-4 bei der Herstellung eines Medikaments für die Behandlung von Vogelgrippe.

## Revendications

1. Phage recombinant isolé et purifié portant un peptide de fusion qui contient la séquence d'acides aminés NDFRSKT et se lie au virus de la grippe aviaire sous-type H9N2 et inhibe la propagation de ce dernier.

2. Peptide synthétique qui contient la séquence CNDFRSKTC ou NDFRSKT et inhibe la propagation du virus de la grippe aviaire H9N2 en se liant au virus.

3. Peptide synthétique selon la revendication 2, où ledit peptide possède une valeur IC₅₀ de moins de 100 µm.

4. Peptide synthétique selon l'une quelconque des revendications 2 ou 3, qui se lie aux glycoprotéines de surface HA et NA du virus de la grippe aviaire H9N2.

5. Composition pharmaceutique contenant un phage recombinant selon la revendication 1.

6. Composition pharmaceutique contenant un peptide synthétique selon l'une quelconque des revendications 2-4.

7. Réactif diagnostique contenant un phage recombinant selon la revendication 1.

8. Réactif diagnostique contenant un peptide synthétique selon l'une quelconque des revendications 2-4.

9. Phage recombinant ou peptide synthétique selon l'une quelconque des revendications 1-4, pour une utilisation dans une méthode de traitement ou de diagnostic d'une grippe aviaire.

10. Utilisation d'un phage recombinant ou d'un peptide synthétique selon l'une quelconque des revendications 1-4 dans la fabrication d'un médicament pour le traitement d'une grippe aviaire.
